# EUROPEAN PATENT APPLICATION

(11) **EP 3 109 627 A1**
(43) Date of publication of application: **28.12.2016**
(21) Application number: 15751674.1
(22) Date of filing: 20.02.2015
(51) Int. Cl.: G01N 27/00, C12M 1/00, C12M 1/34

(54) **ELECTRODES FOR BIOMOLECULAR SEQUENCING DEVICE, AND BIOMOLECULAR SEQUENCING DEVICE, METHOD, AND PROGRAM**

(30) Priority: 20.02.2014 JP 2014031084
(71) Applicant: Quantum Biosystems Inc., 2-3-11, Nihonbashihoncho, Chuo-ku, Tokyo 103-0023 (JP)
(72) Inventor: KAWAI, Tomoji, Suita-shi, Osaka 565-0871 (JP); TANIGUCHI, Masateru, Suita-shi, Osaka 565-0871 (JP); OHSHIRO, Takahito, Suita-shi, Osaka 565-0871 (JP)
(74) Representative: Grünecker Patent- und Rechtsanwälte PartG mbB
(86) International application number: PCT/JP2015/054796
(87) International publication number: WO 2015/125920

(57) **Abstract**

The nano-gap electrode pair 12 is disposed so that a biomolecule joined to at least one or more types of a single molecule included in a sample passes an opposing position, and the strength of the electric field in a position spaced only a predetermined distance on the downstream side from the opposing position 64 becomes stronger than the strength of the electric field in a position spaced only the predetermined distance on the upstream side from the opposing position 64.

## Description

### Field of the Invention

The present invention relates to an electrode for a biomolecular sequencing device, a biomolecular sequencing device, method, and program.

### Background of the Invention

Conventionally, sequencing is carried out to determine a sequence for a single molecule configuring a biomolecule, such as an amino acid sequence configuring a protein, a nucleotide sequence configuring a nucleic acid, and a monosaccharide sequence configuring a sugar chain, and especially for a biopolymer. For example, protein sequences are determined using an HPLC (high performance liquid chromatography) method using a method of enzymatic decomposition, mass spectrometry, X-ray crystallography, the Edman degradation method, and the like.

Further, a sequencing technique has been proposed for identifying a single molecule by measuring the tunnel current flowing from one molecule using nano-gap electrodes having a fixed inter-electrode distance of 1nm or less. Further, various techniques of this type have been proposed relating to the flow path from a sample (for example see patent documents 1, and 2).

### Prior Art Documents

### Patent Documents

Patent Document 1: Japanese Unexamined Patent Application No. 2010-227735
Patent Document 2: Japanese Unexamined Patent Application No. 2012-110258

### Summary of the Invention

### Problems to be Solved by the Invention

A unimolecular electrical measuring method that identifies a single molecule through the tunnel current can identify a single molecule by directly measuring the electronic energy of a molecular sample. There is a problem in conventional unimolecular electrical measuring methods wherein the frequency at which a single molecule passes between sensing electrodes is low and the degree of accuracy for detecting a single molecule is low because the probability of an accidental event due to Brownian motion through thermal fluctuation is used in the methods for introducing a molecular sample.

Methods for introducing a molecular sample in a solution include methods using pump pressure or electroosmotic flow, but both are insufficient to resolve the low frequency at which molecules pass between sensing electrodes because neither can induce a steady flow that can be controlled on a molecular scale. Therefore, a problem with the conventional unimolecular electrical measuring method through tunnel current is that application is only possible when the objective is resequencing under limited conditions such as when using a solution having a high concentration of a pure sample.

The present invention was made in light of the problems described above, and an object thereof is to provide an electrode for a biomolecular sequencing device, a biomolecular sequencing device, method, and program capable of identifying a single molecule composing a biomolecule with a high degree of accuracy.

### Means to Resolve the Problems

In order to achieve the objective describe above, the electrode for a biomolecular sequencing device according to the present invention is provided with an electrode pair disposed so that tunnel current flows when a biomolecule joined to at least one or more types of a single molecule included in a sample passes the opposing position, and the shape of the downstream side is differentiated from the shape of the upstream side of the electrode pair so that the strength of the electric field in a position spaced only a predetermined distance on the downstream side from the opposing position becomes stronger than the strength of the electric field in a position spaced only the predetermined distance on the upstream side from the opposing position.

According to the present invention, the shape of the downstream side is differentiated from the shape of the upstream side of the electrode pair so that the strength of the electric field in a position spaced only a predetermined distance on the downstream side from the opposing position of the electrode becomes stronger than the strength of the electric field in a position spaced only the predetermined distance on the upstream side from the opposing position. That is, the shape of the electrode pair is asymmetric, the upstream side being the introduction side of the biomolecule and the downstream side being the discharged side of the biomolecule. By this a stable and steady flow can be induced by promoting an electrophoretic force of a biomolecule in a sample, and a single molecule can be identified with a high level of precision because stable electrophoresis of a biomolecule can be carried out.

It should be noted that the shape of the upstream flow path on the upstream side gradually widens as separation from the opposing position increases upstream, and the shape of the downstream flow path on the downstream side gradually widens as separation from the opposing position increases downstream, and the shape of the downstream side may be differentiated from the shape of the upstream side of the electrode pair so that the widening angle of the downstream flow path becomes even smaller than the widening angle of the upstream flow path.

Further, the shape of the downstream side may be differentiated from the shape of the upstream side of the electrode pair so that the length of the downstream flow path in the direction orthogonal to the opposing direction of the electrode pair becomes even longer than the length of the upstream flow path in the orthogonal direction.

Further, the length of the downstream flow path in an orthogonal direction is preferably no less than two times and no more than four times as long as the upstream flow path in the orthogonal direction.

Further, the shape of the downstream side may be differentiated from the shape of the upstream side of the electrode pair so that the length of the arc formed by the intersection of a circle centralized on the center, the narrowest point between the electrode pair, and the end of the downstream flow path becomes shorter than the arc formed by the intersection of the circle and the end of the upstream flow path.

Further, the shape of the downstream side may be differentiated from the shape of the upstream side of the electrode pair so that the area of the downstream flow path on the downstream side included in a predetermined range centralized on the center, the narrowest point between the electrode pair, becomes smaller than the area of the upstream flow path on the upstream side included in the predetermined range.

Further, the plurality of electrode pairs may each have a different inter-electrode distance.

The biomolecular sequencing device according to the present invention is provided with the electrode for a biomolecular sequencing device, a measuring part that measures tunnel current that occurs when the biomolecule passes the opposing position of the electrode pairs of the electrode for a biomolecular sequencing device, and an identifying part that identifies a variety of at least one type of single molecule composing the biomolecule based on a detected physical quantity obtained from the tunnel current measured by the measuring part.

The biomolecular sequencing method executed in the biomolecular sequencing device provided with the electrode for the biomolecular sequencing device according to the present invention measures tunnel current that occurs when the biomolecule passes the opposing position of the electrode pairs of the electrode for the biomolecular sequencing device, and identifies a variety of at least one type of single molecule composing the biomolecule based on a detected physical quantity obtained from the measured tunnel current.

The biomolecular sequencing program according to the present invention enables a computer to function as the measuring part and the identifying part of the biomolecular sequencing device.

### Effect of the Invention

According to the present invention, a single molecule composing a biomolecule can be identified with a high degree of accuracy.

### Brief Description of the Drawings

FIG. 1 is a schematic drawing depicting a configuration of the biomolecular sequencing device according to embodiment 1.
FIG. 2 is an enlarged drawing around the nano-gap electrode pair in embodiment 1.
FIG. 3 is an enlarged drawing of one part of FIG. 3.
FIG. 4 is a diagram for describing the electric field that occurs when a voltage is applied to the nano-gap electrode pair.
FIG. 5 is a diagram depicting the measurement results for a read time when the length of the upstream flow path and the length of the downstream flow path of the nano-gap electrode pair have a 1:1 ratio.
FIG. 6 is a diagram depicting the measurement results for a read time when the length of the upstream flow path and the length of the downstream flow path of the nano-gap electrode pair have a 1:2 ratio.
FIG. 7 is a diagram depicting the measurement results for a read time when the length of the upstream flow path and the length of the downstream flow path of the nano-gap electrode pair have a 1:4 ratio.
FIG. 8 is a diagram depicting the relationship of the measurement results for the read time and the length of the downstream flow path with respect to the length of the upstream flow path of the nano-gap electrode pair.
FIG. 9 is a block diagram depicting a functional configuration of the controlling part in embodiment 1.
FIG. 10 is a flowchart depicting the biomolecular sequencing process in embodiment 1.
FIG. 11 is a graph depicting the read count for each length of base read.
FIG. 12 is a graph depicting the measurement results of the read count for each percentage of the number of times of a normal read with respect to the number of times a read was transitioned.
FIG. 13 is a schematic drawing depicting a configuration of the biomolecular sequencing device according to embodiment 2.
FIG. 14 is a block diagram depicting a functional configuration of the controlling part in embodiment 2.
FIG. 15 is a flowchart depicting the biomolecular sequencing process in embodiment 2.
Embodiments of the Present Invention

Below, embodiments of the present invention are described in detail with reference to drawings. The following embodiment is described for a case in which a biomolecule is sequenced by measuring the tunnel current that flows when a single molecule passes between electrodes.

### <Embodiment 1>

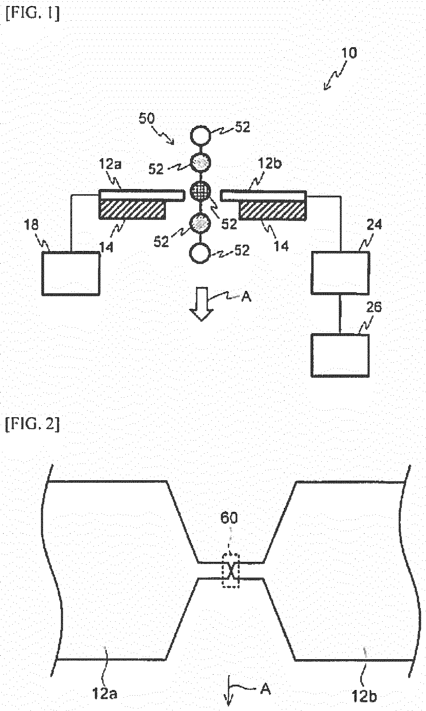

As depicted in FIG. 1, a biomolecular sequencing device 10 according to embodiment 1 includes and is composed of a nano-gap electrode pair 12 (12a, 12b) as the electrode for the biomolecular sequencing device, a measurement power supply 18, an ammeter 24, and a controlling part 26. Each configuration is described below.

The nano-gap electrode pair 12 is composed of two opposing nano-gap electrodes 12a and 12b. The nano-gap electrodes 12a and 12b are disposed at such a distance that tunnel current flows when a single molecule 52 composing a biomolecule included in a sample 50 flows in the direction depicted by arrow A in FIG. 1 and passes between the electrodes. Here a biopolymer such as a protein, peptide, nucleic acid, or sugar chain is included in the biomolecule. Further, an amino acid composing a protein or peptide, a nucleotide composing a nucleic acid, a monosaccharide composing a sugar chain, or the like are included in the single molecule composing the biomolecule, but are not limited thereto.

If the inter-electrode distance is much longer than the molecular diameter of the single molecule 52, it is difficult for the tunnel current to flow between the nano-gap electrode pair 12, and two or more of the single molecule 52 will fit between the nano-gap electrode pair 12 at the same time. Meanwhile, if the inter-electrode distance is much shorter than the molecular diameter of the single molecule 52, the single molecule 52 will not fit between the nano-gap electrode pair 12.

If the inter-electrode distance is much longer or much shorter than the molecular diameter of the single molecule 52, it is difficult to detect the tunnel current relating to the single molecule 52. Therefore, it is preferable for the inter-electrode distance to be slightly shorter than, equal to, or slightly longer the molecular diameter of the single molecule 52. For example, the inter-electrode distance is 0.5 to 2 times as long as the molecular diameter of the single molecule 52, where 1 to 1.5 times as long is preferable, and I to 1.2 times as long is more preferable.

The specific manufacturing method for the nano-gap electrode pair 12 is not particularly limited. One example of a manufacturing method is described below.

The nano-gap electrode pair 12 can be manufactured by using a known method for nanofabricated mechanically-controllable break junctions. The nanofabricated mechanically-controllable break junction method is excellent and can control mechanically stable, excellent inter-electrode distances at a resolution of a picometer or lower. A manufacturing method for an electron pair using the nanofabricated mechanically-controllable break junction method is described, for example, in J.M. van Ruitenbeek, A. Alvarez, I. Pineyro, C. Grahmann, P. Joyez, M.H. Devoret, D. Esteve, C. Urbina, Rev. Sci. Instrum. 67. 108 (1996) or M. Tsutsui, K. Shoji, M. Taniguchi, T. Kawai, Nano Lett. 8, 345 (2008). Examples of the electrode material include any metal such as gold.

For example, the nano-gap electrode pair 12 can be manufactured by the process described below.

First, a bridge of gold on a nano scale is patterned onto a flexible metal substrate coated with polyimide by a known electron beam lithography and lift-off technique using an electron beam lithography device (manufactured by JEOL Ltd., catalog number: JSM6500F). Then the polyimide under the junction is removed by etching based on a known etching method (such as reactive ion etching) using a reactive ion etching device (SAMCO Inc., catalog number: 10NR).

Then a bridge of gold on a nano scale, having a bent structure in three points, is manufactured by bending the substrate. In this case, the inter-electrode distance of the electrode pair can be controlled at a resolution of a picometer or lower by precisely controlling the bending of the substrate using a piezo actuator (CEDRAT Co., catalog number: APA150M).

Then tension is applied to the manufactured bridge and one part of the bridge is fractured. Further tension is applied to the bridge, and the size of the gap (inter-electrode distance), which occurs as a result of the fracture, is set to the length of an objective single molecule 52. For example, when the single molecule 52 is an amino acid molecule composing a peptide disassembled from a protein, which is a biopolymer, into an appropriate length, the length thereof is approximately 1nm. In this case the inter-electrode distance of the electrode pair can be precisely controlled by adjusting the bridge tension using a self-fracturing technology (see M. Tsutsui, K. Shoji, M. Taniguchi, T. Kawai, Nano Lett. 8, 345 (2008), and M. Tsutsui, M. Taniguchi, T. Kawai, Appl. Phys. Lett. 93, 163115 (2008)).

Specifically, 0.1 V of DC bias voltage (Vb) is applied to the bridge using a series of 10kΩ resistance under a programmed juncture elongation speed through a resistance feedback method (see M. Tsutsui, K. Shoji, M. Taniguchi, T. Kawai, Nano Lett. 8, 345 (2008), and M. Tsutsui, M. Taniguchi, T. Kawai, Appl. Phys. Lett. 93, 163115 (2008)) using a data acquisition board (National Instruments Corp., catalog number: NIPCIe-6321), tension is applied to the gold nano joint, and the bridge is fractured. Then further tension is applied to the bridge, and the size of the gap (inter-electrode distance), which occurred through the fracture, is set to an objective length. By this the nano-gap electrode pair 12 is formed.

The measurement power supply 18 applies a voltage to the nano-gap electrode pair 12. The size of the voltage applied to the nano-gap electrode pair 12 by the measurement power supply 18 is not particularly limited, and for example 0.25V to 0.75V can be applied. The specific configuration of the measurement power supply 18 is not particularly limited, and a known power supply device can be used as appropriate.

The ammeter 24 measures the tunnel current that occurs when the single molecule 52 passes between the electrodes of the nano-gap electrode pair 12, to which a voltage is applied by the measurement power supply 18. The specific configuration of the ammeter 24 is not particularly limited, and a known current measuring device may be used as appropriate.

Next the specific configuration around the nano-gap electrode 12 of the biomolecular sequencing device 10 is described.

An enlarged plan view of the surroundings between the electrodes of the nano-gap electrode pair 12 is depicted in FIG. 2. As depicted in FIG. 2, the nano-gap electrodes 12a and 12b have a bilaterally symmetric shape, and each end part narrows.

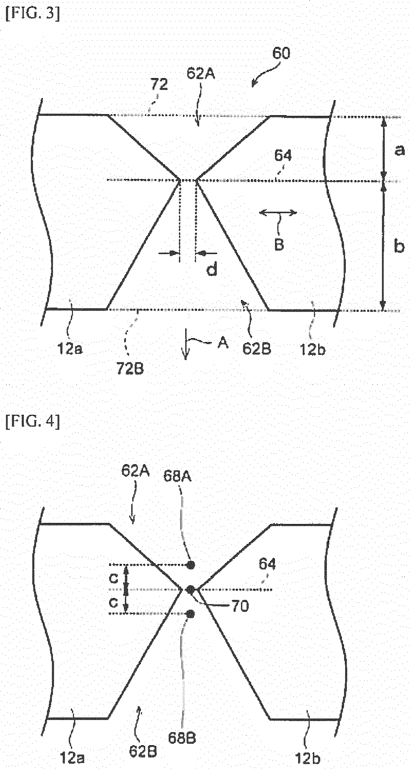

An enlarged plan view of a region 60, depicted by a dotted line in FIG. 2, is depicted in FIG. 3. It is preferable for an inter-electrode distance d of the nano-gap pair 12 to be slightly shorter than, equal to, or slightly longer than the molecular diameter of the single molecule 52, being, for example, several hundred pm to 1.0nm.

Further, as depicted in FIG. 3, the end parts of the nano-gap electrodes 12a and 12b have a bilaterally symmetric shape, and the shape of the downstream side is differentiated from the shape of the upstream side of the nano-gap electrode pair 12 as the strength of the electric field in the positions spaced only a predetermined distance downstream from an opposing position 64, the narrowest point between electrodes, becomes stronger than the strength of the electric field in a position spaced only the predetermined distance upstream from the opposing position 64. Here the shape of the upstream side is the shape of the side in FIG. 3 above the opposing position 64, and the shape of the downstream side is the shape of the side in FIG. 3 below the opposing position 64.

Specifically, the shape of an upstream flow path 62A on the upstream side in the flow path for the sample 50 gradually widens as separation from the opposing position 64 increases upstream, and the shape of a downstream flow path 62B on the downstream side gradually widens as separation from the opposing position 64 increases downstream, and the shape of the downstream side is differentiated from the shape of the upstream side of the nano-gap electrodes 12a and 12b as the widening angle of the downstream flow path 62B becomes even smaller than the widening angle of the upstream flow path 62A.

Herewith the density of the electrical line of force in the electric field formed in the downstream flow path 62B becomes stronger than the density of the electrical line of force in the electric field formed in the upstream flow path 62A when a voltage is applied to the nano-gap electrodes 12a and 12b.

Therefore, as depicted in FIG. 4, the strength of the electric field in a position 68B separated only a predetermined distance c downstream from the opposing position 64 becomes stronger than the strength of the electric field in a position 68A separated only the same predetermined distance c upstream from the opposing position 64. Herewith a stable, steady flow can be induced by promoting the electrophoretic force of a biomolecule included in the sample 50, and the single molecule 52 can be identified with a high level of precision because stable electrophoresis of a biomolecule can be carried out.

It should be noted that if the shape of the nano-gap electrode pair 12 is one wherein the shape of the downstream side is differentiated from the upstream side as the strength of the electric field in the position 68B spaced only the predetermined distance c downstream from the opposing position 64 becomes stronger than the strength of the electric field in the position 68A spaced only the same predetermined distance c upstream from the opposing position 64, the shape is not limited to that depicted in FIG. 3,4.

Further, the nano-gap electrode pair 12 depicted in FIG. 4 can also be one wherein the shape of the downstream side is differentiated from the upstream side as the length of the arc formed by the intersection of a circle centralized on a center 70, the narrowest point between the electrodes, and an end of the downstream flow path 62B (the end of the downstream side of the nano-gap electrode pair 12) becomes shorter than the arc formed by the intersection of the circle and the end of the upstream flow path 62A (the end of the upstream side of the nano-gap electrode pair 12).

Further, the shape of the downstream side is differentiated from the upstream side of the nano-gap electrode pair 12 depicted in FIG. 4 as the area of the downstream flow path 62B included in a predetermined range centralized on the center 70, the narrowest point between the electrodes, becomes smaller than the area of the upstream flow path 62A included in the predetermined range. Here the predetermined range is portrayed as a symmetrical shape such as a circle, square, or rectangle.

Further, as depicted in FIG. 3, the shape of the downstream side is differentiated from the upstream side of the nano-gap electrode pair 12 as the length b of the downstream flow path 62B in the orthogonal direction A orthogonal to the opposing direction B of the electrodes 12a and 12b becomes even longer than the length a of the upstream flow path 62A in the orthogonal direction. Here the length a of the upstream flow path 62A is the distance from the opposing position 64 to an end part 72A of the upstream side of the nano-gap electrode pair 12, and the length of the downstream flow path 62B is the distance from the opposing position 64 to an end part 72B of the downstream side of the nano-gap electrode pair 12.

And it is preferable that a length b of the downstream flow path 62B in the orthogonal direction A is no less than two times the length a of the upstream flow path 62A in the orthogonal direction A.

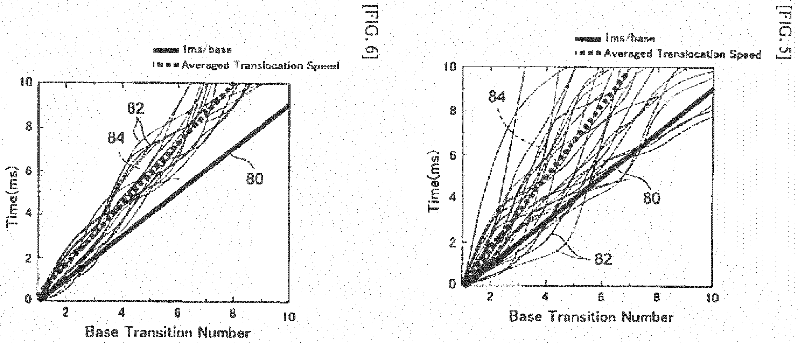

FIG. 5 depicts a graph of the base read times, as done conventionally, for when the shape of the downstream side and the shape of the upstream side of the nano-gap electrode pair 12 are the same, that is when the length a of the upstream flow path 62A and the length b of the downstream flow path 62B have a 1:1 ratio. A thick line 80 in the graph is a straight line portraying the ideal base read time, which is approximately 1ms for each base. Further, a plurality of thin lines 82 in the graph portray the read time for each base, and a dotted line 84 portrays the average value for the plurality of thin lines 82. Here the variability of the read time can be said to be small the closer the plurality of thin lines 82 get to the dotted line 84, and can be said to be close to the ideal read time the closer the dotted line 84 gets to the thick line 80, but as depicted in FIG. 5, the variability of the plurality of thin lines 82 is large and the dotted line 84 is separated from the thick line 80 using a conventional configuration.

A graph of the base read times when the shape of the downstream side and the shape of the upstream side are differentiated is depicted in FIG. 6 for the nano-gap electrode pair 12 according to the present embodiment. FIG. 6 depicts a graph of the base read times when the length a of the upstream flow path 62A and the length b of the downstream flow path 62B have a 1:2 ratio. As depicted in FIG. 6, the plurality of thin lines 82 are closer to the dotted line 84, and the variability of the read time can be said to be small when compared to the case in FIG. 5 using a conventional configuration.

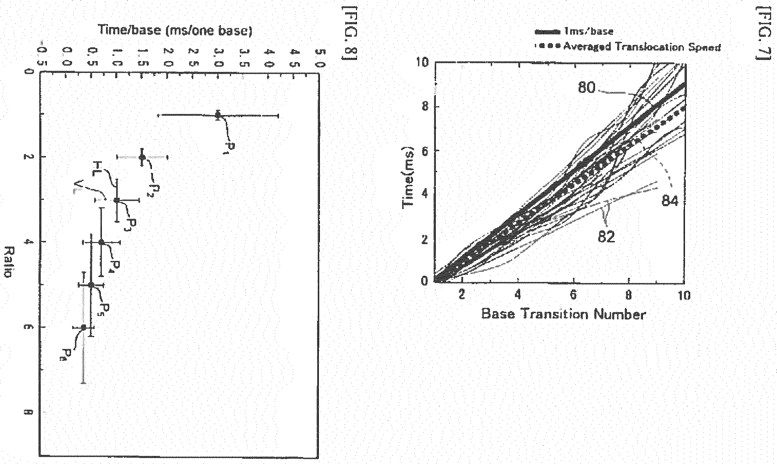

Further, FIG. 7 depicts a graph of the base read times when the length a of the upstream flow path 62A and the length b of the downstream flow path 62B have a 1:4 ratio. As depicted in FIG. 7, the plurality of thin lines 82 are closer to the dotted line 84, and the variability of the read time can be said to be small when compared to the case in FIG. 5 wherein a conventional configuration is used. Further, the dotted line 84 is closer to the thick line 80, and the read time can be said to be close to the ideal.

Further, FIG. 8 depicts the measured results of the read times for a nucleic acid base chain when the length a of the upstream flow path 62A and the length b of the downstream flow path 62B have a 1:Ratio ratio. It should be noted that the measurement results in FIG. 8 are measured at a measurement acquisition rate of 10kHz for the signal corresponding to the tunnel current. Further, the PRatio in the drawing is plotted in the position where each average value for the read times and Ratio are portrayed. Further, a vertical line VL centered on the PRatio depicts the range of measured read times (variation), and a horizontal line HL depicts the range of measured Ratios (variation). The read times ideally have small variation, in the vicinity of 1 ms, but as depicted in FIG. 8 the average value for the read times approaches 1ms when the Ratio=2 to 4, and is preferable because the variation is small.

As described above, the length of the downstream flow path 62B is preferably no less than two times and no more than four times as long as the length a of the upstream flow path 62A in the orthogonal direction A.

The controlling part 26 controls each configuration in the biomolecular sequencing device 10, and identifies the type of single molecule 52 based on a signal corresponding to a measured tunnel current.

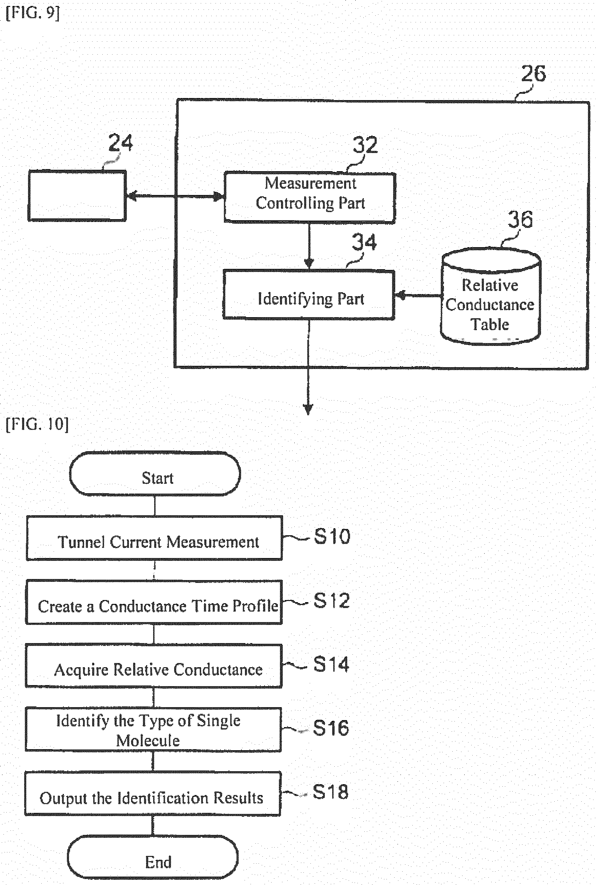

The controlling part 26 can be composed of a computer provided with a CPU (Central Processing Unit), RAM (Random Access Memory), and ROM (Read Only Memory) stored by the biomolecular sequencing program explained hereafter, and the like. The controlling part 26 composed of this computer can be functionally portrayed by a configuration including a measurement controlling part 32 and an identifying part 34 as depicted in FIG. 9. Each part is described in detail below.

The measurement controlling part 32 controls the ammeter 24 as tunnel current flowing between the electrodes of the nano-gap electrode pair 12 is measured. The measurement time for tunnel current is not limited, but for example 10 minute, 20 minute, 30 minute, 40 minute, 50 minute, and one hour periods can be carried out. The measurement time is arbitrarily set according to the length of the single molecule 52.

Further, the measurement controlling part 32 acquires a current value for tunnel current measured by the ammeter 24, calculates the conductance from the acquired current value, and creates a conductance time profile. Conductance can be calculated by dividing the current value for a tunnel current by the Voltage V applied to the nano-gap electrode pair 12 when the tunnel current is measured. By using the conductance, a unified, standard profile can be obtained even if each measured voltage value applied between the nano-gap electrode pair 12 is different. It should be noted that the current value of a tunnel current and conductance can be treated equivalently when the voltage value applied between the nano-gap electrode pair 12 in each measurement is fixed.

Further, the measurement controlling part 32 may be made to acquire tunnel current measured by the ammeter 24 after one amplification using a current amplifier. Tunnel current can be measured at a high degree of sensitivity because a weak tunnel current value can be amplified using a current amplifier. For example, a commercially available variable high-speed current amplifier (Femto, Inc., catalog number: DHPCA-100) can be used as the current amplifier.

The identifying part 34 can identify the type of single molecule 52 by comparing a detected physical quantity obtained from the conductance time profile created by the measurement controlling part 32, with the relative conductance for the single molecule 52 of a known type stored in a relative conductance table 36. In the present embodiment, the detected physical quantity has a conductance for each measurement point of the conductance time profile created by the measurement controlling part 32. Here there is a relative conductance for each type of single molecule 52 measured by a known type of single molecule 52, and is calculated by dividing the conductance for one molecule of each single molecule 52 by the maximum conductance value measured for each type of single molecule 52.

Next the function of the biomolecular sequencing device 10 according to embodiment 1 is described.

First, at least one type of the single molecule 52 targeted for identification is dissolved in a solution. The solution is not particularly limited. For example ultrapure water can be used. Ultrapure water can be manufactured, for example, using the Millipore Corp. Milli-Q Integral 3 (device name) (Milli-Q Integral 3/5/10/15 (catalog number)). The concentration of the single molecule 52 in the solution is not particularly limited, but for example 0.01 to 1.0µM is possible.

Now the nano-gap electrode pair 12 is disposed in a sample and a voltage is applied to the nano-gap electrode pair 12 by the measurement power supply 18. Now the biomolecular sequencing process depicted in FIG. 10 is carried out by the biomolecular sequencing device 10 wherein the biomolecular sequencing program stored on the ROM is read and executed by the CPU of the computer composing the controlling part 26.

In a step S10, the measurement controlling part 32 controls the ammeter 24, and the tunnel current that occurs when the single molecule 52 passes between the electrodes of the nano-gap electrode pair 12 is measured at a prescribed time.

Next, in a step S12, the measurement controlling part 32 acquires a current value for the measured tunnel current, calculates the conductance at each measurement point, and creates a conductance time profile.

Next, in a step S14, the identifying part 34 acquires the relative conductance of a single molecule 52 targeted for identification from the relative conductance table 36.

Next, in a step S16, the identifying part 34 compares the conductance time profile created by the step S12 and the relative conductance acquired by the step S14, and identifies the type of single molecule depicted by each signal. Next, in a step S18, the identifying part 34 outputs the identification results and terminates the single molecule identification process.

As described above, because the shape of the downstream side is differentiated from the shape of the upstream side of the nano-gap electrode pair 12 as the strength of the electric field downstream from the opposing position 64 becomes stronger than the strength of the electric field upstream according to the biomolecular sequencing device according to embodiment 1, a stable, steady flow can be induced by promoting the electrophoretic force of a biomolecule included in the sample 50, and the single molecule 52 can be identified with a high level of precision because stable electrophoresis of a biomolecule can be carried out.

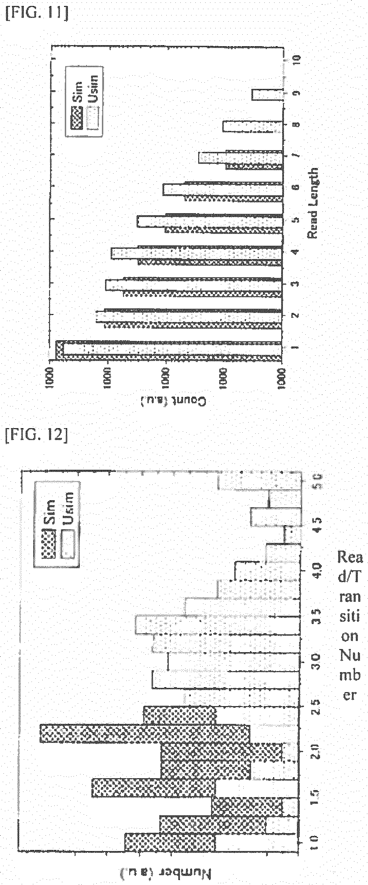

FIG. 11 depicts the measured read count results for each base length read both for when the shape on the upstream side of the nano-gap electrode pair 12 and the shape on the downstream side are symmetrical (Sim), as is conventional, and for when the shape on the upstream side of the nano-gap electrode pair 12 and the shape on the downstream side are unsymmetrical (Usim), as in the present embodiment. As depicted in FIG. 11, it can be seen that when the shape on the upstream side of the nano-gap electrode pair 12 and the shape on the downstream side are unsymmetrical, as in the present embodiment, the overall read count increases.

Further, FIG. 12 depicts the measured results for the read count for each percentage of the number of times of a normal read with respect to the number of times of a read transition in which the migration direction of a base transitioned both for when the nano-gap electrode pair 12 has a conventional configuration (Sim) and for the present embodiment (Usim), similar to the description above. As depicted in FIG. 12, the larger the values on the horizontal axis, the less frequently a read transition occurs, and it can be seen that the read transitions for the present invention are decreased and the frequency of successful reads is high compared to the conventional configuration.

By this, it can be seen that the read count can be increased and the read transitions can be decreased through the shape of the upstream side and the shape of the downstream side being unsymmetrical, as in the nano-gap electrode pair 12 according to the present embodiment.

### <Embodiment 2>

Next, embodiment 2 is described. It should be noted that parts identical to the biomolecular sequencing device 10 according to embodiment 1 use identical reference numerals but descriptions thereof are omitted.

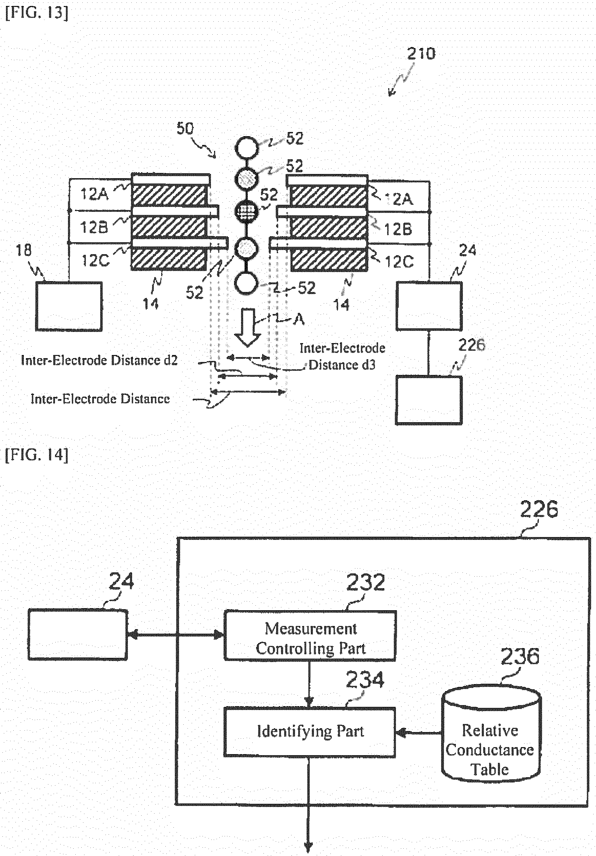

As depicted in FIG. 13, a biomolecular sequencing device 210 according to embodiment 2 includes and is composed of nano-gap electrodes 12A, 12B, and 12C, the measurement power supply 18, ammeter 24, and a controlling part 226.

The configuration of each nano-gap electrode 12a, 12B, and 12C is similar to the nano-gap electrode pair 12 in embodiment 1. Each nano-gap electrode 12A, 12B, and 12C is layered through an insulator 14 so that the center between each electrode is arranged on the same axis. That is, a single passage for the single molecule 52 is formed between each electrode of the nano-gap electrodes 12A, 12B, and 12C. The inter-electrode distance d1 for the nano-gap electrode 12a, the inter-electrode distance d2 for the nano-gap electrode 12B, and the inter-electrode distance d3 for the nano-gap electrode 12C are each different. In the example in FIG. 13, d1>d2>d3. For example, d1=1.0nm, d2=0.7nm, and d3=0.5nm is possible.

The controlling part 226 can be portrayed by a configuration provided with a measurement controlling part 232 and an identifying part 234 as depicted in FIG. 14.

The measurement controlling part 232 controls the ammeter 24 as each tunnel current that occurs between each of the nano-gap electrodes 12a, 12B, and 12C is measured. Further, the measurement controlling part 232 calculates the conductance by acquiring the current value for the tunnel current of each inter-electrode distance measured by the ammeter 24, and creates a conductance time profile for each inter-electrode distance.

The identifying part 234 can identify the type of single molecule 52 by comparing a detected physical quantity obtained from the conductance time profile created by the measurement controlling part 32 with the relative conductance for the single molecule 52 of a known type stored in a relative conductance table 236.

Next the function of the biomolecular sequencing method, carried out using the biomolecular sequencing device 210 according to embodiment 2, is described.

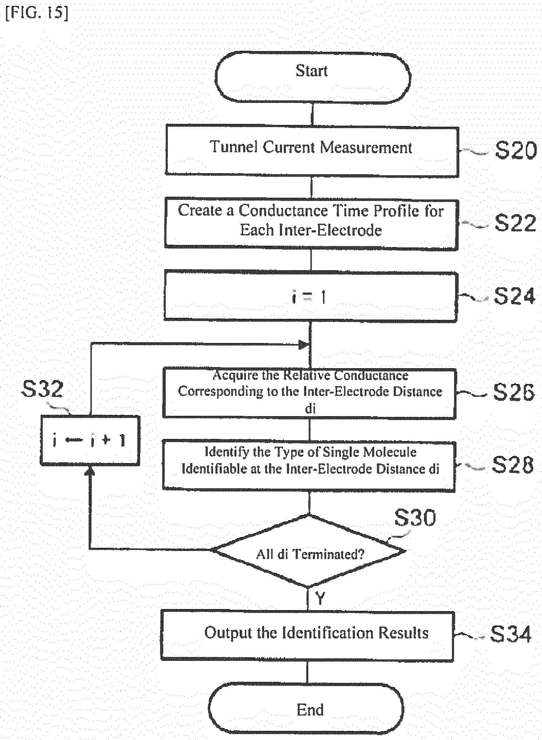

First, at least one type of the single molecule 52 targeted for identification is dissolved in a solution similar to embodiment 1. Now the nano-gap electrodes 12A, 12B, and 12C are disposed in a sample and a voltage is applied to each of the nano-gap electrodes 12A, 12B, and 12C by the measurement power supply 18. Now the biomolecular sequencing process depicted in FIG. 15 is carried out by the biomolecular sequencing device 210 wherein the biomolecular sequencing program stored on the ROM is read and executed by the CPU of the computer composing the controlling part 226.

In a step S20, the measurement controlling part 232 controls the ammeter 24, and the tunnel current that occurs when the single molecule 52 passes through the single passage formed between each of the nano-gap electrodes 12A, 12B, and 12C is measured at a prescribed time.

Next, in a step S22, the measurement controlling part 232 acquires a current value for the measured tunnel current, calculates the conductance at each measurement point, and creates a conductance time profile for each inter-electrode distance.

Next, in a step S24, the identifying part 234 sets a 1 for the variable number i.

Next, in a step S26, the identifying part 234 acquires the relative conductance for the single molecule 52 corresponding to the inter-electrode distance di, that is, the relative conductance for the single molecule 52 targeted for identification, targetable according to the inter-electrode distance di, from the relative conductance table 236.

Next, in a step S28, the identifying part 234 compares the conductance time profile for the inter-electrode distance di created by the step S22 and the relative conductance acquired by the step S26, and identifies the type of single molecule depicted by each signal.

Next, in a step S30, the identifying part 234 determines whether the processes for all of the inter-electrode distances di have been terminated. When an inter-electrode distance di for a remaining process exists, step S32 is switched to, a 1 is incremented for i, and it returns to step S26. When all processes for all of the inter-electrode distances di have been terminated, it switches to step S34 and the identifying part 234 outputs the identification results and terminates the biomolecular sequencing process.

As described above, in addition to the effect of embodiment 1, identification can be carried out with a higher degree of precision using conductance obtained from the tunnel current that occurs between the nano-gap electrodes of the plurality of inter-electrode distances according to the biomolecular sequencing device according to embodiment 2.

Further, a case wherein a plurality of nano-gap electrode pairs having different inter-electrode distances is described in embodiment 2, but a configuration may be provided with a mechanism that converts the inter-electrode distance of one nano-gap electrode pair. For example, using this principle a configuration can be made to convert an inter-electrode distance by adjusting the geometric disposition of the force point, fulcrum point, and point of application. More specifically, a configuration can be made to move the electrode end, being the point of application, and convert the inter-electrode distance by pushing a part of the nano-gap electrode pair up using a piezo element. In this case, a desired inter-electrode distance can be set based on the corresponding relationship between the distance pushed up by the piezo element and the inter-electrode distance.

Further, the present invention is not limited to each configuration described by each embodiment described above, a wide variety of changes are possible according to the scope described in the scope of patent claims, and an embodiment obtained by combining any technical means disclosed in the different embodiments respectively is included in the technical scope of the present invention.

Further, an embodiment having a pre-installed program is described in the present specification, but a program loaded on an external storage device, recording medium, or the like may be read as needed, or a program may be executed by downloading through the internet. Further, the program can be provided by being loaded onto a recording medium capable of being read by a computer.

### Description of Symbols

10, 210 Biomolecular sequencing device
12, 12A, 12B, 12C Nano-gap electrode pair
18 Measurement power supply
24 Ammeter
26, 226 Controlling part
32, 232 Measurement controlling part
34, 234 Identifying part
36, 236 Relative conductance table
50 Sample
52 Single molecule

## Claims

1. An electrode for a biomolecular sequencing device, comprising:
an electrode pair disposed so that tunnel current flows when a biomolecule joined to at least one or more types of a single molecule included in a sample passes an opposing position,
wherein a shape of a downstream side is differentiated from a shape of an upstream side of the electrode pair so that a strength of an electric field in a position spaced only a predetermined distance on the downstream side from the opposing position becomes stronger than a strength of an electric field in a position spaced only the predetermined distance on the upstream side from the opposing position.

2. The electrode for a biomolecular sequencing device according to claim 1, wherein a shape of an upstream flow path on the upstream side gradually widens as separation from the opposing position increases on the upstream side, and a shape of a downstream flow path on the downstream side gradually widens as separation from the opposing position increases on the downstream side, and a shape of the downstream side is differentiated from a shape of the upstream side of the electrode pair so that the widening angle of the downstream flow path becomes even smaller than the widening angle of the upstream flow path.

3. The electrode for a biomolecular sequencing device according to claim 2, wherein a shape of the downstream side is differentiated from a shape of the upstream side of the electrode pair so that a length of the downstream flow path in a direction orthogonal to an opposing direction of the electrode pair becomes even longer than a length of the upstream flow path in the orthogonal direction.

4. The electrode for a biomolecular sequencing device according to claim 3, wherein a length of the downstream flow path in the orthogonal direction is no less than two times and no more than four times as long as a length of the upstream flow path in the orthogonal direction.

5. The electrode for a biomolecular sequencing device according to any one of claims 1 to 4, wherein a shape of the downstream side is differentiated from a shape of the upstream side of the electrode pair so that a length of an arc formed by the intersection of a circle centralized around the center of the narrowest point between the electrode pair and an end of the downstream flow path becomes shorter than a length of an arc formed by the intersection of the circle and an end of the upstream flow path on the upstream side.

6. The electrode for a biomolecular sequencing device according to any one of claims I to 5, wherein a shape of the downstream side is differentiated from a shape of the upstream side of the electrode pair so that an area of the downstream flow path on the downstream side included in a predetermined range centralized around the center of the narrowest point between the electrode pair becomes smaller than an area of the upstream flow path on the upstream side included in the predetermined range.

7. The electrode for a biomolecular sequencing device according to any one of claims 1 to 6, wherein a plurality of electrode pairs each have a different inter-electrode distance.

8. A biomolecular sequencing device, comprising:
the electrode for a biomolecular sequencing device according to any one of claims 1 to 7;
a measuring part that measures tunnel current that occurs when the biomolecule passes an opposing position of the electrode pairs of the electrode for a biomolecular sequencing device; and
an identifying part that identifies a variety of at least one type of single molecule composing the biomolecule based on a detected physical quantity obtained from the tunnel current measured by the measuring part.

9. A biomolecular sequencing method executed in the biomolecular sequencing device provided with the electrode for the biomolecular sequencing device according to any one of claims 1 to 7, comprising:
measuring tunnel current that occurs when the biomolecule passes an opposing position of the electrode pairs of the electrode for the biomolecular sequencing device; and
identifying a variety of at least one type of single molecule composing the biomolecule based on a detected physical quantity obtained from the measured tunnel current.

10. A biomolecular sequencing program that enables a function as a measuring part and an identifying part of the biomolecular sequencing device according to claim 8 function.
